# EUROPEAN PATENT APPLICATION

(11) **EP 4 772 640 A1**
(43) Date of publication of application: **08.07.2026**
(21) Application number: 24857874.2
(22) Date of filing: 24.05.2024
(51) Int. Cl.: C12N 15/64, A61K 48/00, C12P 19/34

(54) **PREPARATION METHOD FOR MINICIRCLE DNA AND USE OF MINICIRCLE DNA**

(30) Priority: 30.08.2023 CN 202311106818
(71) Applicant: Triarm Therapeutics (Shanghai) , Ltd., Shanghai 200120 (CN)
(72) Inventor: WENG, Jingjie, Shanghai 200120 (CN); LU, Jinhua, Shanghai 200120 (CN); ZHU, Hong, Shanghai 200120 (CN); LU, Dexin, Shanghai 200120 (CN); ZHANG, Jay, Shanghai 200120 (CN)
(74) Representative: Lavoix
(86) International application number: PCT/CN2024/095317
(87) International publication number: WO 2025/044328

(57) **Abstract**

Provided are a preparation method for a minicircle DNA and a use of the minicircle DNA. Specifically, provided is a recombinant plasmid, which carries a CinH resolvase and a specific recognition site therefor, and is used for generating a minicircle DNA. Further provided are a method for generating a minicircle DNA by using the recombinant plasmid, a kit comprising the recombinant plasmid and used for generating a minicircle DNA, and a use of the minicircle DNA, which is prepared by using the method, in gene therapy and cell therapy.

## Description

### TECHNICAL FIELD

The present invention relates to the field of biomedicine, and in particular to a preparation method of minicircle DNA, as well as its application in gene therapy and cell therapy.

### BACKGROUND

DNA vectors are widely used in vaccines, gene therapy, cell therapy and other fields. Traditional DNA vectors are plasmids carrying a prokaryotic replication origin and an antibiotic resistance gene. However, these prokaryote-derived DNA sequences may inhibit the expression of cis-linked mammalian cell expression cassettes, stimulate the DNA sensing pathway, activate T lymphocytes, B lymphocytes, NK cells and monocytes to promote immune inflammatory responses, and induce programmed cell death. All these responses limit the application efficacy of plasmid DNA vectors. In addition, the spread of prokaryotic replication origins and antibiotic resistance genes is also one of the risks associated with the use of plasmid DNA vectors.

Supercoiled circular DNA from which the prokaryotic replication origin and antibiotic resistance gene had been removed was first prepared by Darquet et al. in 1997, and exhibited superior protein expression capacity to plasmid DNA vectors in a variety of mammalian cells. This type of DNA is referred to as minicircle DNA. Chen et al. further used mice as a model to demonstrate that when DNA vectors were delivered to the mouse liver, minicircle DNA enabled the expression of proteins with clinical therapeutic potential in the peripheral blood at levels 200 to 560 times higher than those of plasmid DNA. Since then, the high stability and high-efficiency expression capacity of minicircle DNA have been widely used in the development of gene therapy or cell therapy for various diseases. Minicircle DNA has been tested as a gene therapy vector in preclinical studies for rheumatoid arthritis or osteoarthritis. Rim et al. used minicircles to express BMP2 and TGFβ3, induced the differentiation of mesenchymal stem cell-like cells derived from hiPSCs into chondrocytes, and successfully treated osteochondral injuries in a rat model. Florian et al. expressed Angiopoietin 1 protein in mesenchymal stem cells using minicircle DNA, which enhanced the efficacy of mesenchymal stem cells in alleviating acute lung injury. Technologies for delivering proteins with clinical therapeutic potential to retinal epithelial cells, retinal ganglion cells or photoreceptor cells for the treatment of eye-related diseases using minicircle DNA are also under development. Overall, minicircle DNA is a vector for gene therapy and cell therapy with high potential and a wide range of applications.

The preparation of minicircle DNA relies on recombinases to split a parental plasmid carrying two recombination sites into two smaller circular DNAs. Through the design of the relative positions of the recombination sites with respect to other elements on the parental plasmid, one of the circular DNAs can be made to carry an expression cassette of a functional protein or functional RNA required for gene therapy or cell therapy, namely the minicircle DNA, while the other circular DNA carries the elements to be removed, including the prokaryotic replication origin and antibiotic resistance gene, and is generally referred to as a miniplasmid. The recombinases reported to be applicable for preparing minicircle DNA at present include bacteriophage λ integrase, bacteriophage ΦC31 integrase, Cre recombinase, ParA resolvase and the like. Minicircles prepared with bacteriophage λ integrase, ΦC31 integrase and Cre recombinase are all contaminated with dimers or multimers. Since these dimers and multimers have the identical sequence to the target minicircle DNA, this increases the difficulty of obtaining high-purity minicircle DNA in subsequent steps. In contrast, ParA resolvase features a recombination efficiency close to 100%, and the products after recombination are free of dimer or multimer contamination. The acquisition of high-purity minicircles also requires the combination of technologies for removing residual parental plasmids and miniplasmids. The common methods at present include *in vitro* enzymatic digestion for removing parental plasmids and miniplasmids, *in vivo* enzymatic digestion for removing parental plasmids and miniplasmids, affinity chromatography for removing parental plasmids and miniplasmids, or purification of minicircle DNA and the like. Whole-column chromatography has also been applied for the further purification of supercoiled minicircle DNA to meet the standards for clinical application.

Adoptive cellular immunotherapy has achieved remarkable outcomes in recent years. Following the approval of KYMRIAH (Novartis Pharmaceuticals Corporation), YESCARTA and TECARTUS (Kite Pharma, Inc.), BREYANZI (Juno Therapeutics, Inc.), ABECMA (Celgene Corporation), CARVYKTI (Janssen Biotech, Inc.), etc., by the U.S. Food and Drug Administration (FDA) for the clinical treatment of relapsed and refractory hematologic malignancies, including large B-cell lymphoma, mantle cell lymphoma, multiple myeloma, etc., a novel and reliable treatment strategy has been provided to prolong patients' survival and improve their quality of life. All these adoptive immunotherapies approved by the FDA are prepared using retroviral or lentiviral vectors. For clinically used CAR-T cells prepared with retroviral vectors and lentiviral vectors, 65% and 54% of the insertion sites are located within RefSeq genes, respectively. The insertion of exogenous genes into these RefSeq genes has raised concerns about the application of retroviral and lentiviral vectors in gene therapy and cell therapy. The Sleeping Beauty transposon system has been proven to insert into the human genome in a nearly random manner, and exhibits higher safety for gene therapy applications compared with retroviral, lentiviral and other transposon system-based vectors. However, the major obstacle to the application of non-viral vector systems in gene therapy or cell therapy is the low delivery efficiency of the vectors.

In contrast, minicircle DNA can significantly improve the efficiency of non-viral DNA vectors owing to its characteristics such as high stability and high expression level. Therefore, minicircle DNA based on the Sleeping Beauty transposon system, which carries the CAR gene for the preparation of CAR-T cells, can effectively replace the high-risk retroviral or lentiviral vectors.

Thus, there is an enormous demand in the art for the research and development of minicircle DNA medicaments.

### SUMMARY OF THE INVENTION

The objective of the present invention is to provide a recombinant plasmid which carries elements including a prokaryotic replication origin, a prokaryotic selection marker, a regulatable expression element controlling recombinase expression, a recombinase, two recombinase recognition sites, a multiple cloning site for inserting a target DNA sequence, and the like, and is used for producing circular supercoiled minicircle DNA free of prokaryote-derived DNA sequences such as the prokaryotic replication origin and the prokaryotic selection marker. The present invention also provides a method for producing minicircle DNA using the said recombinant plasmid.

In the first aspect of the present invention, it provides a recombinant plasmid, which comprises the following elements:
a prokaryotic replication origin, a prokaryotic selection marker, a recombinase coding sequence, a regulatable expression element controlling recombinase expression, a multiple cloning site for inserting a target DNA sequence, and two recombinase recognition sites respectively flanking the multiple cloning site;
and the arrangement of the above elements is such that the plasmid can be cleaved by the expressed recombinase at the two recombinase recognition sites into a miniplasmid and a minicircle DNA, wherein the formed minicircle DNA comprises the multiple cloning site for inserting the target DNA sequence, the target DNA sequence inserted therein, and a residual recombinase recognition site;
wherein the recombinase is CinH resolvase, and the recombinase recognition site has a nucleotide sequence as set forth in SEQ ID NO: 3 or a derivative sequence thereof.

In another preferred embodiment, the CinH resolvase comprises wild-type CinH resolvase and mutants thereof, wherein the mutants retain the activity of the CinH resolvase to recognize and cleave the recombinase recognition site as set forth in SEQ ID NO: 3.

In another preferred embodiment, the amino acid sequence of the CinH resolvase is set forth in SEQ ID NO: 1.

In another preferred embodiment, the nucleotide sequence encoding the CinH resolvase is set forth in SEQ ID NO: 2.

In another preferred embodiment, the two recombinase recognition sites flanking the multiple cloning site are in the same orientation, i.e., → multiple cloning site →.

In another preferred embodiment, the target DNA sequence is selected from the group consisting of a functional DNA sequence, a functional RNA expression cassette, a functional protein expression cassette, and a combination thereof.

In another preferred embodiment, the target DNA sequence is a protein expression cassette for expressing a transposase in eukaryotic cells; wherein the transposase includes (but is not limited to) Sleeping Beauty, PiggyBac and To12.

In another preferred embodiment, the target DNA sequence is a transposon, which cooperates with a transposase to stably insert the carried genetic information into the genome of prokaryotic or eukaryotic cells.

In another preferred embodiment, the target DNA sequence comprises a chimeric antigen receptor (CAR) coding sequence.

In another preferred embodiment, the regulatable expression element controlling recombinase expression is selected from the group consisting of the pBAD/AraC system of the arabinose operon, the LacI/LacO system of the lactose/IPTG operon, the rtTA/TRE system of the tetracycline operon, a heat shock-inducible system based on heat shock protein 70 or 90, and a light-inducible system based on the blue light-sensing protein YFI and the downstream FixJ/FixK2/cI/pR thereof.

In another preferred embodiment, the recombinant plasmid further comprises a miniplasmid recognition and removal element, which is located in the miniplasmid produced after the plasmid is cleaved by the expressed recombinase.

In another preferred embodiment, the miniplasmid recognition and removal element is selected from the group consisting of a restriction enzyme cleavage site, a LacO sequence, a specifically designed complementary sequence of oligonucleotides or deoxynucleotides, a specifically designed recognition sequence for dead Cas ribonucleoproteins (such as dead Cas9, Cas12, Cas3, Cas8, Cas10 and Cas13 RNP, and the like), a specifically designed recognition sequence for zinc finger DNA binding domain, a specifically designed recognition sequence for transcription activator-like effector DNA binding domain, and a combination thereof.

In another preferred embodiment, the miniplasmid recognition and removal element is at least one restriction enzyme cleavage site.

In another preferred embodiment, the recombinant plasmid further comprises a minicircle DNA recognition and isolation element, which is located in the minicircle DNA produced after the plasmid is cleaved by the expressed recombinase.

In another preferred embodiment, the minicircle DNA recognition and isolation element is selected from the group consisting of a LacO sequence, a specifically designed complementary sequence of oligonucleotides or deoxynucleotides, a specifically designed recognition sequence for dead Cas ribonucleoproteins (such as dead Cas9, Cas12, Cas3, Cas8, Cas10 and Cas13 RNP, and the like), a specifically designed recognition sequence for zinc finger DNA binding domain, a specifically designed recognition sequence for transcription activator-like effector DNA binding domain, and a combination thereof.

In another preferred embodiment, the recombinant plasmid further comprises a miniplasmid recognition and removal element, and a minicircle DNA recognition and isolation element.

In the second aspect of the present invention, it provides a reaction system for producing minicircle DNA, which comprises:
(Z1) the recombinant plasmid of the first aspect of the present invention;
(Z2) an inducing agent or induction condition for inducing the expression of the recombinase encoded in the recombinant plasmid.

In another preferred embodiment, the inducing agent is selected from the group consisting of arabinose, lactose or IPTG, and tetracycline.

In another preferred embodiment, the induction condition comprises heat shock or light induction.

In another preferred embodiment, the reaction system further comprises a miniplasmid recognition and removal reagent, and/or a minicircle DNA recognition and isolation reagent.

In another preferred embodiment, the miniplasmid recognition and removal reagent acts on the miniplasmid recognition and removal element in the recombinant plasmid and the produced miniplasmid, and is used for removing the miniplasmid and unreacted recombinant plasmid from the product.

In another preferred embodiment, the minicircle DNA recognition and isolation reagent acts on the minicircle DNA recognition and isolation element in the minicircle DNA, and is used for isolating the minicircle DNA from the product.

In another preferred embodiment, the miniplasmid recognition and removal reagent is selected from the group consisting of restriction endonuclease, LacI protein, oligonucleotides or deoxynucleotides of specifically designed sequence, dead Cas ribonucleoproteins (such as dead Cas9, Cas12, Cas3, Cas8, Cas10 and Cas13 RNP and the like), zinc finger DNA binding domain, transcription activator-like effector DNA binding domain, and a combination thereof.

In another preferred embodiment, the miniplasmid recognition and removal reagent is a restriction endonuclease.

In another preferred embodiment, the minicircle DNA recognition and isolation reagent comprises a protein capable of binding to the minicircle DNA recognition and isolation element in the minicircle DNA to form a stable DNA-protein complex; optionally, the protein is immobilized on a solid phase carrier.

In another preferred embodiment, the minicircle DNA recognition and isolation reagent is selected from the group consisting of LacI protein, oligonucleotides or deoxynucleotides of specifically designed sequence, dead Cas ribonucleoproteins (such as dead Cas9, Cas12, Cas3, Cas8, Cas10 and Cas13 RNP and the like), zinc finger DNA binding domain, transcription activator-like effector DNA binding domain, and a combination thereof.

In the third aspect of the present invention, it provides a kit for producing minicircle DNA, which comprises:
(C1) the recombinant plasmid of the first aspect of the present invention; and
(C2) optionally, an inducing agent for inducing the expression of the recombinase encoded in the recombinant plasmid.

In another preferred embodiment, the kit further comprises:
(C3) a miniplasmid recognition and removal reagent, which acts on the miniplasmid recognition and removal element in the recombinant plasmid and the produced miniplasmid, and is used for removing the miniplasmid and unreacted recombinant plasmid from the product; and/or
(C4) a minicircle DNA recognition and isolation reagent, which acts on the minicircle DNA recognition and isolation element in the minicircle DNA, and is used for isolating the minicircle DNA from the product.

In another preferred embodiment, the inducing agent is selected from the group consisting of arabinose, lactose or IPTG, and tetracycline.

In another preferred embodiment, the miniplasmid recognition and removal reagent is selected from the group consisting of restriction endonuclease, LacI protein, oligonucleotides or deoxynucleotides of specifically designed sequence, dead Cas ribonucleoproteins (such as dead Cas9, Cas12, Cas3, Cas8, Cas10 and Cas13 RNP and the like), zinc finger DNA binding domain, transcription activator-like effector DNA binding domain, and a combination thereof.

In another preferred embodiment, the miniplasmid recognition and removal reagent is a restriction endonuclease.

In another preferred embodiment, the minicircle DNA recognition and isolation reagent comprises a protein capable of binding to the minicircle DNA recognition and isolation element in the minicircle DNA to form a stable DNA-protein complex; optionally, the protein is immobilized on a solid phase carrier.

In another preferred embodiment, the minicircle DNA recognition and isolation reagent is selected from the group consisting of LacI protein, oligonucleotides or deoxynucleotides of specifically designed sequence, dead Cas ribonucleoproteins (such as dead Cas9, Cas12, Cas3, Cas8, Cas10 and Cas13 RNP and the like), zinc finger DNA binding domain, transcription activator-like effector DNA binding domain, and a combination thereof.

In another preferred embodiment, the kit further comprises a label or instructions, which describe a method of producing minicircle DNA using the kit, wherein the method comprises inducing the expression of the recombinase in the recombinant plasmid in the presence of an inducing agent or an induction condition.

In another preferred embodiment, the induction condition comprises heat shock or light induction.

In the fourth aspect of the present invention, it provides a method for producing minicircle DNA, which comprises:
(S1) introducing the recombinant plasmid of the first aspect of the present invention into a prokaryotic cell;
(S2) culturing the prokaryotic cell and inducing the expression of the recombinase encoded in the recombinant plasmid, thereby producing an intermediate product comprising a miniplasmid and minicircle DNA; and
(S3) isolating minicircle DNA from the intermediate product of (S2).

In another preferred embodiment, in step (S2), the regulatable expression element controlling recombinase expression in the recombinant plasmid is activated by adding an inducing agent, thereby inducing recombinase expression.

In another preferred embodiment, in step (S2), the regulatable expression element controlling recombinase expression in the recombinant plasmid is activated by use of an induction condition, thereby inducing recombinase expression.

In another preferred embodiment, in step (S3), the minicircle DNA is isolated with a minicircle DNA recognition and isolation reagent by utilizing the minicircle DNA recognition and isolation element in the minicircle DNA.

In another preferred embodiment, in step (S3), the miniplasmid and unreacted recombinant plasmid in the intermediate product are removed with a miniplasmid recognition and removal reagent, thereby isolating the minicircle DNA.

In another preferred embodiment, in step (S3), the miniplasmid and unreacted recombinant plasmid in the intermediate product are first removed with a miniplasmid recognition and removal reagent, and then the minicircle DNA is isolated with a minicircle DNA recognition and isolation reagent by utilizing the minicircle DNA recognition and isolation element in the minicircle DNA.

In another preferred embodiment, the prokaryotic cell includes (but is not limited to) *Escherichia coli*, *Agrobacterium tumefaciens*, *Bacillus subtilis*, *Lactobacillus, Salmonella, Bifidobacterium, Listeria monocytogenes, Pseudomonas, Neisseria meningitidis*, and *Vibrio cholerae*.

In the fifth aspect of the present invention, it provides a minicircle DNA prepared by the method of the fourth aspect of the present invention, which comprises:
a multiple cloning site for inserting a target DNA sequence, the target DNA sequence inserted therein, and a residual recombinase recognition site; wherein the recombinase recognition site has a nucleotide sequence as set forth in SEQ ID NO: 3 or a derivative sequence thereof.

In another preferred embodiment, the target DNA sequence is selected from the group consisting of a functional DNA sequence, a functional RNA expression cassette, a functional protein expression cassette, and a combination thereof.

In another preferred embodiment, the target DNA sequence is a protein expression cassette for expressing a transposase in eukaryotic cells; the transposase includes (but is not limited to) Sleeping Beauty, PiggyBac, and To12.

In another preferred embodiment, the target DNA sequence is a transposon that cooperates with a transposase to stably insert the carried genetic information into the genome of a prokaryotic or eukaryotic cell.

In another preferred embodiment, the target DNA sequence comprises a chimeric antigen receptor (CAR) coding sequence.

In another preferred embodiment, the minicircle DNA further comprises a minicircle DNA recognition and isolation element.

In another preferred embodiment, the minicircle DNA recognition and isolation element is selected from the group consisting of a LacO sequence, a specifically designed complementary sequence of oligonucleotides or deoxynucleotides, a specifically designed recognition sequence for dead Cas ribonucleoproteins, a specifically designed recognition sequence for zinc finger DNA binding domain, a specifically designed recognition sequence for transcription activator-like effector DNA binding domain, and a combination thereof.

In the sixth aspect of the present invention, it provides a pharmaceutical composition comprising:
(a) the minicircle DNA of the fifth aspect of the present invention; and (b) a pharmaceutically acceptable carrier.

In another preferred embodiment, the pharmaceutical composition is used for the treatment and/or prevention of diseases.

In another preferred embodiment, the pharmaceutical composition is used for gene therapy or cell therapy.

In another preferred embodiment, the pharmaceutical composition is used as a vaccine for the treatment and/or prevention of diseases.

In the seventh aspect of the present invention, it provides a use of the recombinant plasmid of the first aspect of the present invention in the preparation of a minicircle DNA drug.

In another preferred embodiment, the minicircle DNA drug is used for gene therapy or cell therapy.

In the eighth aspect of the present invention, it provides a use of the minicircle DNA of the fifth aspect of the present invention in the preparation of a medicament for the treatment and/or prevention of diseases.

In another preferred embodiment, the disease can be treated by gene therapy or cell therapy.

In another preferred embodiment, the disease includes (but is not limited to) cancer or tumor, ocular disease, rheumatoid arthritis, osteoarthritis, osteochondral injury and acute lung injury.

In the ninth aspect of the present invention, it provides a method for the treatment and/or prevention of a disease, which comprises administering to a subject in need thereof the minicircle DNA of the fifth aspect of the present invention.

In another preferred embodiment, the minicircle DNA acts as a delivery vector to deliver a therapeutic/prophylactic ingredient into the subject.

In another preferred embodiment, the subject includes a human or a non-human mammal.

In another preferred embodiment, the disease can be treated by gene therapy or cell therapy.

In another preferred embodiment, the disease includes (but is not limited to) cancer or tumor, ocular disease, rheumatoid arthritis, osteoarthritis, osteochondral injury and acute lung injury.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### DESCRIPTION OF THE DRAWINGS

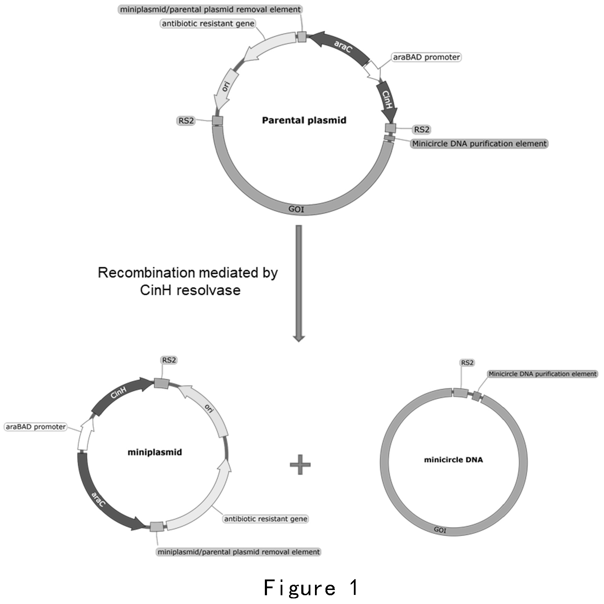

Figure 1 shows a schematic diagram of the element arrangement and configuration of the minicircle DNA parental plasmid, the miniplasmid, and the minicircle DNA.
Figure 2a shows the preparation process of the transposon minicircle carrying an EGFP expression cassette.
Figure 2b shows the successful preparation of the transposon minicircle DNA carrying an EGFP expression cassette.
Figure 3 shows the preparation of the transposon minicircle DNA carrying a CD19-CAR expression cassette.
Figure 4 shows the preparation of the minicircle DNA carrying a transposase expression cassette.
Figure 5 shows that minicircle DNA is significantly superior to plasmid DNA when used as a delivery vector to deliver exogenous genes into T cells.

### DETAILED DESCRIPTION

After extensive and in-depth research, the present inventors have developed a novel recombinant plasmid for the first time. The arrangement of the various elements comprised in the recombinant plasmid provided by the present invention enables the parental plasmid to be split into two smaller circular DNAs upon induced expression of the recombinase; one of the resulting DNAs is a miniplasmid comprising non-target sequences such as a prokaryotic replication origin, a prokaryotic selection marker, a regulatable expression element controlling recombinase expression, and a recombinase coding sequence; the other is minicircle DNA comprising only the target sequence, a residual multiple cloning site, and a residual recombinase recognition site. The miniplasmid can be removed by means of a specific restriction enzyme site, a specific recognition sequence, or the like on the miniplasmid to purify the minicircle DNA; alternatively, the minicircle DNA can be purified via a specific recognition sequence on the minicircle DNA.

On the basis of the above, the present invention has been completed.

### Term

For a better understanding of the present invention, certain technical and scientific terms are specifically defined herein. Unless expressly defined otherwise herein, all other technical and scientific terms used herein have the meanings that are commonly understood by one of ordinary skill in the art to which this invention belongs. Before describing the present invention, it is to be understood that this invention is not limited to the specific methods and experimental conditions described, as such methods and conditions may vary. It is also to be understood that the term used herein is for the purpose of describing particular embodiments only and is not intended to be limiting, and the scope of the present invention is to be limited only by the appended claims.

Unless otherwise defined, all technical and scientific terms used herein have the same meaning as commonly understood by one of ordinary skill in the art to which this invention belongs.

As used herein, the term "about" when used in reference to a specifically recited value means that the value may vary by no more than 1% from the recited value. For example, as used herein, the expression "about 100" includes all values between 99 and 101 (e.g., 99.1, 99.2, 99.3, 99.4, and the like).

The three-letter codes and single-letter codes for amino acids used in the present invention are as described in J. Biol. Chem., 243, p3558 (1968).

As used herein, the terms "optional" or "optionally" mean that the subsequently described event or circumstance may, but need not, occur. For example, "optionally, an inducing agent for inducing the expression of the recombinase in the recombinant plasmid" means that the inducing agent may be present but is not necessarily required.

The term "sequence identity" as used in the present invention refers to the degree of identity between two nucleic acid or two amino acid sequences when optimally aligned and compared with appropriate mutations such as substitutions, insertions or deletions. The sequence identity between a sequence described in the present invention and a sequence identical thereto may be at least 85%, 90% or 95%, preferably at least 95%. Non-limiting examples include 85%, 86%, 87%, 88%, 89%, 90%, 91%, 92%, 93%, 94%, 95%, 96%, 97%, 98%, 99%, 100%.

### The recombinant plasmid of the present invention

In one aspect of the present invention, it provides a recombinant plasmid comprising the following elements: a prokaryotic replication origin, a prokaryotic selection marker, a recombinase coding sequence, a regulatable expression element controlling recombinase expression, a multiple cloning site for inserting a target DNA sequence, and two recombinase recognition sites respectively flanking the multiple cloning site; the arrangement of the above elements enables the plasmid to be cleaved by the expressed recombinase at the two recombinase recognition sites into a miniplasmid and a minicircle DNA; the formed minicircle DNA comprises the multiple cloning site for inserting a target DNA sequence, the target DNA sequence inserted therein, and a residual recombinase recognition site; and the miniplasmid comprises non-target sequences such as a prokaryotic replication origin, a prokaryotic selection marker, a regulatable expression element controlling recombinase expression, and a recombinase coding sequence. The recombinase comprised in the recombinant plasmid of the present invention is CinH resolvase, and the corresponding recombinase recognition site has a nucleotide sequence as set forth in SEQ ID NO: 3 or a derivative sequence thereof.

In the present invention, CinH resolvase includes wild-type CinH resolvase and mutants thereof, wherein the mutants retain the activity of CinH resolvase to recognize and cleave the recombinase recognition site as set forth in SEQ ID NO: 3. Preferably, the mutants have at least 85% sequence identity as compared with the amino acid sequence of wild-type CinH resolvase as set forth in SEQ ID NO: 1. In one embodiment of the present invention, the coding sequence of the CinH resolvase has a nucleotide sequence as set forth in SEQ ID NO: 2.

The amino acid sequence of wild-type CinH resolvase (SEQ ID NO: 1)

MKGQKVGYVRVSSVEQNTGRQLEGIEVDRIFVDRASGKNTDRPKFQEM
LNYVREGDRVIVHSMDRFARSLKDLVTEVDKLVKRGIAIQFVKENITFTAQS
TPMDNLMLQLMGAFAQFEREIILERQKEGIKIAAAQGKYKGRVHKLNPDQA
KALLQAWKEGKYSSKVDLAKAFGISRQAVYRYLKQIN*

The coding sequence of the CinH resolvase (SEQ ID NO: 2)

Gtgaaaggccaaaaagtagggtatgtgcgagtgagttcggtcgagcaaaatacagggcgtcaacttgaggga
attgaagtcgaccggatttttgttgaccgtgcttcgggtaaaaataccgaccgaccgaaatttcaagaaatgttgaactatg
tccgggaaggggatagggtgattgtacattccatggatcgttttgcacgaagtctaaaagatttggtcactgaagtagata
aactggtcaaaagagggatcgccatccagtttgtaaaagagaatattacttttactgcccaatccacgccgatggataattt
gatgctgcaactgatgggtgcttttgctcaattcgagcgggaaattatcttagaacgtcagaaagaagggattaagatcg
cagcagctcagggcaaatataaaggtcgtgtccataaattgaatccagatcaagctaaagcattactgcaagcctggaa
agaagggaagtattcatcaaaagttgatctggcaaaagcgtttggtatcagtagacaggctgtctatcggtatttaaaaca
aattaattag

As used herein, the term "recombinase coding sequence" refers to any sequence required for recombinase expression, such as a promoter, a terminator sequence, and the like. The recombinase itself can be any recombinase capable of catalyzing recombination of the parental plasmid at defined recognition sequences into a miniplasmid and an isolatable minicircle DNA. In the present invention, the recombinase specifically refers to CinH resolvase.

As used herein, the terms "recombinase recognition site" and "RS2" can be used interchangeably and each refers to a sequence capable of being recognized by a recombinase. The recombinant plasmid of the present invention harbors two recombinase recognition sites, and the two recombinase recognition site sequences must be arranged on the plasmid in such a manner that the recombinase can recognize and cleave the sequences to produce a miniplasmid and a minicircle DNA. This means that the recombinase recognition sites shall be located on both sides of the multiple cloning site, and the two recombinase recognition sites are in the same orientation, i.e., → multiple cloning site →, so as to generate a minicircle DNA comprising the target DNA sequence inserted into the multiple cloning site. The resulting minicircle DNA may comprise at least part of one and/or the other recombinase recognition site sequence. The two recombinase recognition site sequences can be identical, or derived from the same recombinase recognition sequence. In the present invention, the recombinase recognition site has a nucleotide sequence as set forth in SEQ ID NO: 3 or a derivative sequence thereof.

Recombinase recognition site (SEQ ID NO: 3)

CGTTACTTTGGGGTATACCCTAAAGTTACAATATAAAAGTTCTTAAA
ACTATGTAACATTTAAATGATTTTTAACCATATATAACATGTAACTTTGAT
ATTTAAAGTTTATAATTTACG

The term "multiple cloning site" refers to a site comprising at least two restriction endonuclease sites, and preferably, it comprises a plurality of sites for a variety of restriction endonucleases.

As used herein, the term "target DNA" refers to a DNA sequence that can be inserted into the multiple cloning site of the aforementioned recombinant plasmid, which is comprised in the resulting minicircle DNA, and finally delivered into a subject to exert a biological function. It can be a functional DNA sequence, a functional RNA expression cassette, and a functional protein expression cassette. Preferably, the target DNA is a gene encoding a therapeutically active protein. Since minicircle DNA is primarily intended for therapy, it preferably comprises a gene encoding a therapeutically active protein. For example, a gene encoding a chimeric antigen receptor (CAR) can be inserted into the multiple cloning site of the recombinant plasmid of the present invention, thereby generating minicircle DNA for CAR expression. However, any gene encoding any protein may of course be inserted, in which case the minicircle can be used in detection assays, studies of biochemical pathways, and the like.

The recombinant plasmid of the present invention comprises a regulatable expression element controlling recombinase expression. By providing a regulatory element for controlling recombinase expression, recombinase expression can be repressed or induced, which allows recombinase to be expressed when necessary. Using this system, the plasmid can be produced in large quantities prior to induction of recombinase expression, thereby achieving maximal efficiency in minicircle DNA production. A variety of regulatory elements are well known to those skilled in the art. Preferably, the regulatory element for controlling recombinase expression comprises a strong promoter. This enables sufficient expression of the recombinase to catalyze recombination of the total amount of plasmid present in the sample, thereby achieving a maximal yield of minicircle DNA. In one embodiment of the present invention, the pBAD/AraC system of the arabinose operon is used to control recombinase expression. This control system represses recombinase expression as long as arabinose is absent from the culture system. When arabinose, e.g., L-arabinose, is added to the culture medium, recombinase expression is induced. This is a very simple and efficient method for regulating recombinase expression.

As used herein, "prokaryotic selection marker" can be an antibiotic resistance gene. This is a method well known in the art for selecting bacteria transfected with a plasmid comprising a prokaryotic selection marker, so as to efficiently produce high-copy-number plasmids.

The recombinant plasmid of the present invention further comprises a minicircle DNA and/or miniplasmid identification sequence for identifying and isolating minicircle DNA. Such an identification sequence provides an efficient means of isolating minicircle DNA from miniplasmid, which further improves the efficiency of minicircle DNA production.

A minicircle DNA or miniplasmid identification sequence is any sequence that is present on a minicircle but not on a miniplasmid (or vice versa) and that enables identification and isolation of minicircle DNA (or removal of miniplasmid). In one embodiment of the present invention, the identification sequence is present on the miniplasmid, and is referred to in the present invention as a "miniplasmid recognition and removal element". In another embodiment of the present invention, the identification sequence is present on the minicircle DNA, and is referred to in the present invention as a "minicircle DNA recognition and isolation element". The aforementioned identification sequence may be present on only the miniplasmid or the minicircle DNA, but it is also applicable that the identification sequence is present on both the miniplasmid and the minicircle DNA.

The miniplasmid recognition and removal element can be a specific sequence that is recognized by a specific reagent and then removed from the product. For example, the miniplasmid recognition and removal element can be a restriction endonuclease recognition site, which is cleaved into linear DNA upon recognition by the corresponding restriction endonuclease, and is further digested and removed from the product.

The minicircle DNA recognition and isolation element can be a specific sequence that will bind to a given ligand, thereby complexing the minicircle DNA with the ligand, and then isolating the complex from miniplasmid, cellular components, and the like, e.g., via an immobilized ligand (the ligand is immobilized on a solid phase carrier, such as magnetic beads, agarose beads, agarose columns, and the like).

### The kit of the present invention

The present invention further provides a kit for producing minicircle DNA, which comprises the recombinant plasmid of the first aspect of the present invention.

In one embodiment of the present invention, the kit comprises the following components: the recombinant plasmid of the first aspect of the present invention; an inducing agent for inducing the expression of the recombinase encoded in the recombinant plasmid; and a miniplasmid recognition and removal reagent. In another embodiment of the present invention, the kit comprises the following components: the recombinant plasmid of the first aspect of the present invention; an inducing agent for inducing the expression of the recombinase encoded in the recombinant plasmid; and a minicircle DNA recognition and isolation reagent. In yet another embodiment of the present invention, the kit comprises the following components: the recombinant plasmid of the first aspect of the present invention; an inducing agent for inducing the expression of the recombinase encoded in the recombinant plasmid; and a miniplasmid recognition and removal reagent and a minicircle DNA recognition and isolation reagent.

Among them, the miniplasmid recognition and removal reagent acts on the miniplasmid recognition and removal element in the recombinant plasmid and the produced miniplasmid, and is used for removing the miniplasmid and unreacted recombinant plasmid from the product. In the present invention, the miniplasmid recognition and removal element and the miniplasmid recognition and removal reagent may be selected from the following paired combinations (miniplasmid recognition and removal element / miniplasmid recognition and removal reagent): restriction endonuclease recognition site / restriction endonuclease, LacO sequence / LacI protein, specifically designed complementary sequence of oligonucleotides or deoxynucleotides / oligonucleotides or deoxynucleotides of specifically designed sequence, specifically designed recognition sequence for dead Cas ribonucleoproteins / dead Cas ribonucleoproteins, specifically designed recognition sequence for zinc finger DNA binding domain / zinc finger DNA binding domain, and specifically designed recognition sequence for transcription activator-like effector DNA binding domain / transcription activator-like effector DNA binding domain. In one preferred embodiment of the present invention, the miniplasmid recognition and removal reagent comprises a restriction endonuclease, which recognizes the restriction endonuclease recognition site located on the miniplasmid and unreacted recombinant plasmid, and cleaves the miniplasmid and unreacted recombinant plasmid into linear DNA. Preferably, the reagent further comprises a linear DNA digestion reagent, such as T5 exonuclease, which digests and removes the linear DNA.

The minicircle DNA recognition and isolation reagent acts on the minicircle DNA recognition and isolation element in the minicircle DNA, and is used for isolating the minicircle DNA from the product. In one preferred embodiment, the minicircle DNA recognition and isolation reagent comprises a protein capable of binding to the minicircle DNA recognition and isolation element in the minicircle DNA to form a stable DNA-protein complex; optionally, the protein is immobilized on a solid phase carrier (such as magnetic beads, agarose beads, agarose columns, and the like). In the present invention, the minicircle DNA recognition and removal element and the minicircle DNA recognition and isolation reagent may be selected from the following paired combinations (minicircle DNA recognition and isolation element / minicircle DNA recognition and isolation reagent): LacO sequence / LacI protein, specifically designed complementary sequence of oligonucleotides or deoxynucleotides / oligonucleotides or deoxynucleotides of specifically designed sequence, specifically designed recognition sequence for dead Cas ribonucleoproteins / dead Cas ribonucleoproteins, specifically designed recognition sequence for zinc finger DNA binding domain / zinc finger DNA binding domain, and specifically designed recognition sequence for transcription activator-like effector DNA binding domain / transcription activator-like effector DNA binding domain.

### The method of the present invention

The present invention provides a method for producing minicircle DNA, which employs the constructed recombinant plasmid according to the first aspect of the present invention to produce minicircle DNA.

In one embodiment of the present invention, the method comprises the following steps:
(S1) introducing the recombinant plasmid of the first aspect of the present invention into a prokaryotic cell;
(S2) culturing the prokaryotic cell, then adding an inducing agent (or subjecting to an induction condition) to induce the expression of the recombinase encoded in the recombinant plasmid, wherein the expressed recombinase cleaves the two recombinase recognition sites on the recombinant plasmid, thereby producing an intermediate product comprising a miniplasmid and minicircle DNA;
(S3) adding a miniplasmid recognition and removal reagent to the intermediate product of (S2) to remove the miniplasmid and unreacted recombinant plasmid therein, thereby isolating minicircle DNA.

In another embodiment of the present invention, the method comprises the following steps:
(S1) introducing the recombinant plasmid of the first aspect of the present invention into a prokaryotic cell;
(S2) culturing the prokaryotic cell, then adding an inducing agent (or subjecting to an induction condition) to induce the expression of the recombinase encoded in the recombinant plasmid, wherein the expressed recombinase cleaves the two recombinase recognition sites on the recombinant plasmid, thereby producing an intermediate product comprising a miniplasmid and minicircle DNA;
(S3) adding a minicircle DNA recognition and isolation reagent to the intermediate product of (S2), thereby isolating minicircle DNA.

In yet another embodiment of the present invention, the method comprises the following steps:
(S1) introducing the recombinant plasmid of the first aspect of the present invention into a prokaryotic cell;
(S2) culturing the prokaryotic cell, then adding an inducing agent (or subjecting to an induction condition) to induce the expression of the recombinase encoded in the recombinant plasmid, wherein the expressed recombinase cleaves the two recombinase recognition sites on the recombinant plasmid, thereby producing an intermediate product comprising a miniplasmid and minicircle DNA;
(S3) adding a miniplasmid recognition and removal reagent to the intermediate product of (S2) to remove the miniplasmid and unreacted recombinant plasmid therein, followed by adding a minicircle DNA recognition and isolation reagent, thereby isolating minicircle DNA.

### The minicircle DNA, pharmaceutical composition and application of the present invention

The present invention further provides minicircle DNA prepared by the method of the present invention, which comprises a multiple cloning site for inserting a target DNA sequence, the target DNA sequence inserted therein, and a residual recombinase recognition site; wherein the recombinase recognition site has a nucleotide sequence as set forth in SEQ ID NO: 3 or a derivative sequence thereof.

Among them, the target DNA sequence includes (but is not limited to) a functional DNA sequence, a functional RNA expression cassette, a functional protein expression cassette, and a combination thereof. In one embodiment, the target DNA sequence is a protein expression cassette for expressing a transposase in eukaryotic cells. In another embodiment, the target DNA sequence is a transposon that cooperates with a transposase to stably insert the carried genetic information into the genome of a prokaryotic or eukaryotic cell.

In one preferred embodiment, the minicircle DNA further comprises a minicircle DNA recognition and isolation element. Alternatively, it does not comprise a minicircle DNA recognition and isolation element, in which case the miniplasmid co-produced therewith during its production comprises a miniplasmid recognition and removal element.

The minicircle DNA of the present invention can be used for preparing a pharmaceutical composition. Accordingly, the present invention also provides a pharmaceutical composition comprising a safe and effective amount of the minicircle DNA of the present invention as an active ingredient and a pharmaceutically acceptable carrier.

As used herein, "safe and effective amount" means an amount of the active ingredient sufficient to significantly ameliorate a condition or symptom without causing severe side effects. "Pharmaceutically acceptable carrier" means one or more compatible solid or liquid fillers or gel substances that are suitable for human use and must have sufficiently high purity and sufficiently low toxicity. "Compatible" as used herein means that each component in the composition can be blended with the active ingredient of the present invention and with each other without significantly reducing the pharmaceutical efficacy of the active ingredient.

The composition can be liquid or solid, such as powder, gel, or paste. Preferably, the composition is liquid, and preferably an injectable liquid.

Examples of pharmaceutically acceptable carriers include cellulose and derivatives thereof (such as sodium carboxymethyl cellulose, sodium ethyl cellulose, cellulose acetate, and the like), gelatin, talc, solid lubricants (such as stearic acid, magnesium stearate), calcium sulfate, vegetable oils (such as soybean oil, sesame oil, peanut oil, olive oil, and the like), polyols (such as propylene glycol, glycerin, mannitol, sorbitol, and the like), emulsifiers (such as Tween^{®}), wetting agents (such as sodium lauryl sulfate), colorants, flavoring agents, stabilizers, antioxidants, preservatives, pyrogen-free water, and the like.

The pharmaceutical composition of the present invention can be used for the treatment and/or prevention of diseases, for example, as a gene therapy or cell therapy medicament, or as a vaccine. This depends on the specific type and use of the target DNA sequence inserted into the minicircle DNA. Diseases treatable by gene therapy or cell therapy include (but are not limited to) cancer or tumors, ocular diseases, rheumatoid arthritis, osteoarthritis, osteochondral injury, and acute lung injury.

### The advantages of the present invention include:

The present invention provides a preparation method and a kit for a highly efficient and low-toxicity DNA vector, which can be used in stages such as research and development, preclinical studies, process development, and clinical studies in fields such as gene therapy and cell therapy, thereby facilitating the rapid development of the gene therapy and cell therapy fields.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions, or according to the conditions recommended by the manufacturers. Percentages and parts are calculated by weight unless otherwise stated. The experiments in the examples or test examples of the present invention for which specific conditions are not indicated are generally performed according to conventional conditions, or according to the conditions recommended by the raw material or commodity manufacturers; reagents for which specific sources are not indicated are conventional reagents purchased from commercial sources.

### Example 1 Preparation of EGFP-Expressing Minicircle DNA

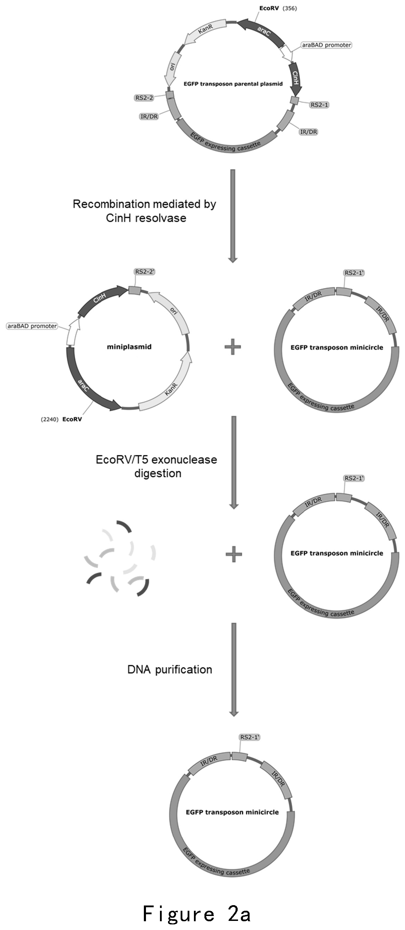

The EGFP expression cassette was designed between two RS sequences. Upon induction of CinH resolvase expression with arabinose to promote plasmid recombination, the miniplasmid can be removed using EcoR V restriction enzyme in combination with T5 exonuclease. After DNA purification, EGFP-expressing minicircle DNA was obtained (Figure 2a).

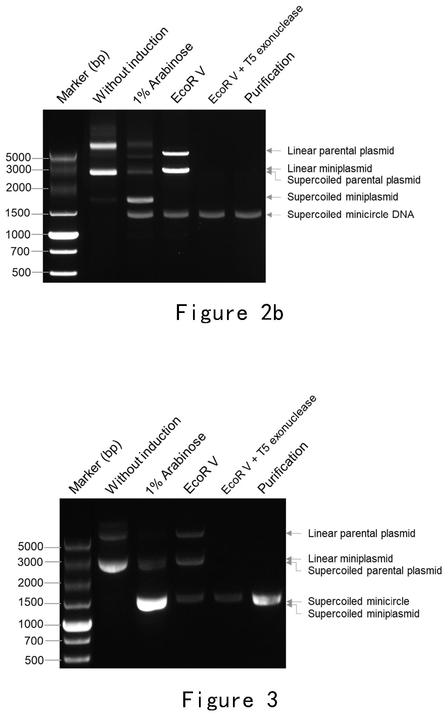

A single colony of *Escherichia coli* harboring the parental plasmid carrying the EGFP expression cassette was inoculated into LB medium and cultured overnight in a constant-temperature incubator at 37 °C with shaking at 200 rpm. After overnight culture, 1% arabinose (BBI, A610071-0100) was added for induction, and culture was continued under the same conditions for 1 hr. The recombinant intermediate DNA product (containing recombined minicircle DNA, miniplasmid DNA, and residual parental plasmid DNA) was then extracted and purified. The specific cleavage site for EcoR V restriction endonuclease was present in the miniplasmid and the parental plasmid, but absent in the minicircle DNA. The intermediate DNA product was treated with EcoR V restriction endonuclease (NEB, R3195L) for 3 hr or overnight to digest the miniplasmid and parental plasmid into linear DNA. T5 exonuclease (NEB, M0663L) was then added and digestion was performed for 3 hr or overnight to remove the linear DNA, leaving only minicircle DNA. The final minicircle DNA was obtained after purification (Figure 2b).

### Example 2 Preparation of Transposon Minicircle DNA Carrying CD19-CAR Expression Cassette

The CD19-CAR expression cassette was constructed into the minicircle-producing parental plasmid. The specific cleavage site for EcoR V restriction endonuclease was present in the recombinant miniplasmid and the parental plasmid, but absent in the minicircle DNA.

A single colony of *Escherichia coli* harboring the parental plasmid carrying the CD19-CAR expression cassette was inoculated into LB medium and cultured overnight in a constant-temperature incubator at 37 °C with shaking at 200 rpm. After overnight culture, 1% arabinose (BBI, A610071-0100) was added for induction, and culture was continued under the same conditions for 1 hr. The recombinant intermediate DNA product (containing recombined minicircle DNA, miniplasmid DNA, and residual parental plasmid DNA) was then extracted and purified. The specific cleavage site for EcoR V restriction endonuclease was present in the miniplasmid and the parental plasmid, but absent in the minicircle DNA. The intermediate DNA product was treated with EcoR V restriction endonuclease (NEB, R3195L) for 3 hr or overnight to digest the miniplasmid and parental plasmid into linear DNA. T5 exonuclease (NEB, M0663L) was then added and digestion was performed for 3 hr or overnight to remove the linear DNA, leaving only minicircle DNA. The final minicircle DNA was obtained after purification (Figure 3).

### Example 3 Preparation of Minicircle DNA Carrying Transposase Expression Cassette

The transposase expression cassette was constructed into the minicircle-producing parental plasmid. The specific cleavage site for EcoR V restriction endonuclease was present in the recombinant miniplasmid and the parental plasmid, but absent in the minicircle DNA.

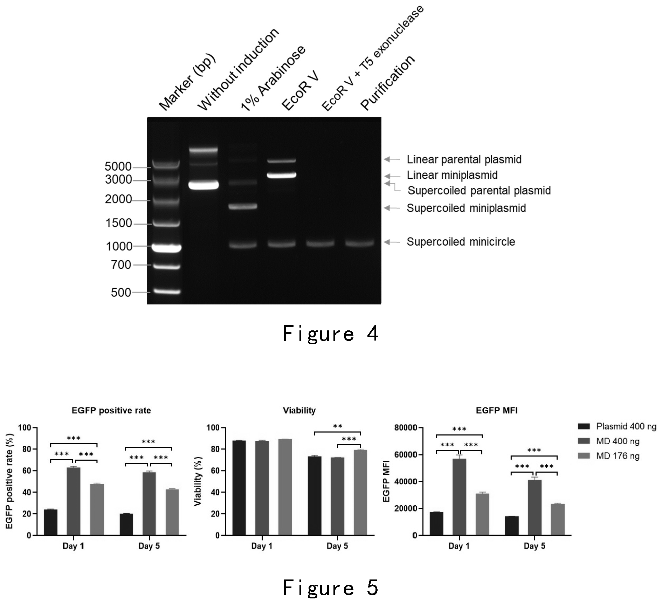

A single colony of *Escherichia coli* harboring the parental plasmid carrying the transposase expression cassette was inoculated into LB medium and cultured overnight in a constant-temperature incubator at 37 °C with shaking at 200 rpm. After overnight culture, 1% arabinose (BBI, A610071-0100) was added for induction, and culture was continued under the same conditions for 1 hr. The recombinant intermediate DNA product (containing recombined minicircle DNA, miniplasmid DNA, and residual parental plasmid DNA) was then extracted and purified. The specific cleavage site for EcoR V restriction endonuclease was present in the miniplasmid and the parental plasmid, but absent in the minicircle DNA. The intermediate DNA product was treated with EcoR V restriction endonuclease (NEB, R3195L) for 3 hr or overnight to digest the miniplasmid and parental plasmid into linear DNA. T5 exonuclease (NEB, M0663L) was then added and digestion was performed for 3 hr or overnight to remove the linear DNA, leaving only minicircle DNA. The final minicircle DNA was obtained after purification (Figure 4).

### Example 4 Minicircle DNA Is Significantly Superior to Plasmid DNA as a Delivery Vector for Delivering Exogenous Genes into T Cells

400 ng of EGFP-expressing plasmid, and an equal mass (400 ng) or equal molar amount (176 ng) of EGFP-expressing minicircle DNA (MD) were delivered into T cells by electroporation (Lonza, AAF-1003X). After electroporation, the cells were cultured in RPMI-1640 medium (Hyclone, SH30027.01) supplemented with 10% FBS (Hyclone, SV30208.02). EGFP positive rate and cell viability were detected by flow cytometry (Agilent, NovoCyte 3110) at 24 hours and 5 days after culture, respectively. 7AAD (BD, 559925) stained positive cells were defined as dead cells. Data are presented as the mean ± SEM of three independent replicates. Significant differences were analyzed by Student's t-test, where ** indicates P < 0.01 and *** indicates P < 0.001.

The results showed that when electroporated at the same mass of DNA, the minicircle DNA group exhibited higher EGFP positive rate and mean fluorescence intensity (MFI) than the plasmid group. When electroporated at the same molar amount, the minicircle DNA group still displayed significantly higher EGFP positive rate, cell viability and MFI than the plasmid group. In the comparison between the two minicircle groups with different dosages, although the low-dose minicircle group was inferior to the high-dose group in EGFP positive rate and MFI, the low-dose group showed significantly better cell viability. The characteristics of minicircle DNA, i.e., lower cytotoxicity and higher expression level compared with plasmid DNA, greatly improve its practicability as a DNA vector for gene therapy or cell therapy (Figure 5).

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. A recombinant plasmid, which comprises the following elements:
a prokaryotic origin of replication, a prokaryotic selection marker, a recombinase coding sequence, a regulatable expression element controlling recombinase expression, a multiple cloning site for inserting a target DNA sequence, and two recombinase recognition sites respectively flanking the multiple cloning site;
and the arrangement of the above elements enables the plasmid to be cleaved by the expressed recombinase at the two recombinase recognition sites into a miniplasmid and a minicircle DNA, wherein the formed minicircle DNA comprises the multiple cloning site for inserting a target DNA sequence, the target DNA sequence inserted therein, and a residual recombinase recognition site;
wherein the recombinase is CinH resolvase, and the recombinase recognition site has a nucleotide sequence as set forth in SEQ ID NO: 3 or a derivative sequence thereof.

2. The recombinant plasmid of claim 1, wherein the CinH resolvase comprises wild-type CinH resolvase and mutants thereof, wherein the mutants retain the activity of CinH resolvase to recognize and cleave the recombinase recognition site as set forth in SEQ ID NO: 3.

3. The recombinant plasmid of claim 1, wherein the target DNA sequence is selected from the group consisting of: a functional DNA sequence, a functional RNA expression cassette, a functional protein expression cassette, and a combination thereof.

4. The recombinant plasmid of claim 1, wherein the target DNA sequence comprises a coding sequence for chimeric antigen receptor (CAR).

5. The recombinant plasmid of claim 1, wherein the regulatable expression element controlling recombinase expression is selected from the group consisting of: the pBAD/AraC system of the arabinose operon, the LacI/LacO system of the lactose/IPTG operon, the rtTA/TRE system of the tetracycline operon, heat shock-inducible systems based on heat shock protein 70 or 90, and light-inducible systems based on the blue light sensor protein YFI and downstream FixJ/FixK2/cI/pR.

6. The recombinant plasmid of claim 1, wherein the recombinant plasmid further comprises: a miniplasmid recognition and removal element, which is located in the miniplasmid produced after the plasmid is cleaved by the expressed recombinase.

7. The recombinant plasmid of claim 6, wherein the miniplasmid recognition and removal element is selected from the group consisting of: a restriction enzyme cleavage site, a LacO sequence, a specifically designed complementary sequence of oligonucleotides or deoxynucleotides, a specifically designed recognition sequence for a dead Cas ribonucleoprotein (such as dead Cas9, Cas12, Cas3, Cas8, Cas10 and Cas13 RNP, and the like), a specifically designed recognition sequence for a zinc finger DNA binding domain, a specifically designed recognition sequence for a transcription activator-like effector DNA binding domain, and a combination thereof.

8. The recombinant plasmid of claim 1, wherein the recombinant plasmid further comprises: a minicircle DNA recognition and isolation element, which is located in the minicircle DNA produced after the plasmid is cleaved by the expressed recombinase.

9. The recombinant plasmid of claim 8, wherein the minicircle DNA recognition and isolation element is selected from the group consisting of: a LacO sequence, a specifically designed complementary sequence of oligonucleotides or deoxynucleotides, a specifically designed recognition sequence for a dead Cas ribonucleoprotein (such as dead Cas9, Cas12, Cas3, Cas8, Cas10 and Cas13 RNP, and the like), a specifically designed recognition sequence for a zinc finger DNA binding domain, a specifically designed recognition sequence for a transcription activator-like effector DNA binding domain, and a combination thereof.

10. A reaction system for producing minicircle DNA, wherein the reaction system comprises:
(Z1) the recombinant plasmid of any one of claims 1 to 9;
(Z2) an inducing agent or an induction condition for inducing the expression of the recombinase encoded in the recombinant plasmid;
in another preferred embodiment, the inducing agent is selected from the group consisting of arabinose, lactose or IPTG, and tetracycline.

11. The reaction system of claim 10, wherein the reaction system further comprises: a miniplasmid recognition and removal reagent, and/or a minicircle DNA recognition and isolation reagent.

12. A kit for producing minicircle DNA, wherein the kit comprises:
(C1) the recombinant plasmid of any one of claims 1 to 9; and
(C2) optionally, an inducing agent for inducing the expression of the recombinase in the recombinant plasmid.

13. The kit of claim 12, wherein the kit further comprises:
(C3) a miniplasmid recognition and removal reagent, which acts on the miniplasmid recognition and removal element in the recombinant plasmid and the produced miniplasmid, and is used for removing the miniplasmid and unreacted recombinant plasmid from the product; and/or
(C4) a minicircle DNA recognition and isolation reagent, which acts on the minicircle DNA recognition and isolation element in the minicircle DNA, and is used for isolating the minicircle DNA from the product.

14. A method for producing minicircle DNA, which comprises:
(S1) introducing the recombinant plasmid of any one of claims 1 to 9 into a prokaryotic cell;
(S2) culturing the prokaryotic cell, and inducing the expression of the recombinase encoded in the recombinant plasmid, thereby producing an intermediate product comprising a miniplasmid and a minicircle DNA; and
(S3) isolating the minicircle DNA from the intermediate product of (S2).

15. A minicircle DNA prepared by the method of claim 14, which comprises:
a multiple cloning site for inserting a target DNA sequence, the target DNA sequence inserted therein, and a residual recombinase recognition site; wherein the recombinase recognition site has a nucleotide sequence as set forth in SEQ ID NO: 3 or a derivative sequence thereof.

16. A pharmaceutical composition, which comprises:
(a) the minicircle DNA of claim 15; and (b) a pharmaceutically acceptable carrier.

17. Use of the recombinant plasmid of any one of claims 1 to 9 in the preparation of a minicircle DNA drug.

18. Use of the minicircle DNA of claim 15 in the preparation of a medicament for the treatment and/or prevention of a disease.
